# DEMANDE DE BREVET EUROPEEN

(11) **EP 1 410 767 A1**
(43) Date de publication de la demande: **21.04.2004**
(21) Numéro de dépôt: 03292540.6
(22) Date de dépôt: 13.10.2003
(51) Int. Cl.: A61B 19/00

(54) **Dispositif à usage unique de transmissions d'informations, utilisé dans les domaines médicaux et chirurgicaux**

(30) Priorité: 16.10.2002 FR 0212877
(71) Demandeur: Promepla Sam, 98000 Monaco (MC)
(72) Inventeur: Danchin, Marc, 06190 Roquebrune Cap Martin (FR)
(74) Mandataire: Wagret, Frédéric

(57) **Abrégé**

La présente invention concerne un dispositif à usage unique de transmissions d'informations permettant, soit l'identification d'un produit circulant, soit la gestion de la distribution/ aspiration d'un produit, aux moyens d'une machine de distribution de produit (3), dans un patient (4), caractérisé en ce qu'il intègre un transpondeur (5) émettant lesdites informations par radiofréquences.

Avantageusement, le dispositif est, soit un outil médical, soit une tubulure (1 ) reliant ladite machine (3) à un patient (4).

## Description

La présente invention concerne un dispositif à usage unique de transmissions d'informations permettant la gestion et le suivi d'un flux d'un produit devant être injecté ou aspiré à un patient lors d'une intervention chirurgicale ou la réalisation de soins médicaux.

Dans le milieu médical, il est indispensable de connaître d'une façon sûre et certaine la nature des produits que l'on injecte au patient et leurs modalités/ conditions d'injection.

Selon l'art antérieur, les informations sur le produit injecté ou aspiré du patient, lors d'une opération par exemple, telles que le numéro de lot de fabrication de produit ou le nom du produit, sont connues grâce à un cahier de procédure que le personnel médical doit lui-même remplir.

Ces informations sont également inscrites directement sur la poche du produit mais cela suppose tout de même leur transcription manuelle sur un support (informatique ou papier).

Dans tous les cas, une manipulation est nécessaire pour enregistrer ces informations, pour pouvoir ainsi les conserver.

Par ailleurs, une même machine de distribution ou d'aspiration de produit est utilisée pour différents types d'opérations chirurgicales, ou de réalisation de soins médicaux.

De ce fait, une telle machine gère, de manière adaptée à l'intervention, une quantité, un débit ou une pression déterminée de produit. Elle doit donc être programmée manuellement avant chaque utilisation.

Par conséquent, une programmation incorrecte peut avoir des conséquences dramatiques sur le patient.

Il convient donc de sécuriser la programmation de la machine de distribution ou d'aspiration de produit de manière à n'autoriser que le réglage adapté au type d'intervention en cours.

Il serait particulièrement avantageux de réaliser un dispositif permettant l'enregistrement de manière automatique d'informations relatives au produit, ainsi que le réglage automatique de la machine de distribution ou d'aspiration de produit.

L'objet de la présente invention se présente sous la forme d'un dispositif à usage unique de transmissions d'informations permettant, soit l'identification d'un produit circulant, soit la gestion de la distribution/aspiration d'un produit, aux moyens d'une machine de distribution ou d'aspiration de produit, dans un patient, caractérisé en ce qu'il intègre un transpondeur émettant lesdites informations par radiofréquences.

De manière avantageuse, le dispositif est, soit un outil à usage médical, notamment chirurgical, soit une tubulure reliant ladite machine de distribution de produit à un patient, ledit produit étant un fluide, par exemple un gaz.

Afin de sécuriser le mode de fonctionnement de la machine de distribution ou d'aspiration du produit, le dispositif selon l'invention transmet, lors de son utilisation, à un récepteur de radiofréquences, lesdites informations.

Avantageusement, le dispositif intègre également un capteur mesurant par exemple la pression, la température, le volume ou le débit du produit circulant en son sein.

Avantageusement, lesdites informations sont transmises, par le récepteur de radiofréquences, à un gestionnaire électronique de la machine qui peut ainsi modifier, sans intervention manuelle, les réglages de la machine de distribution ou d'aspiration du produit.

Afin de conserver un support des différentes informations transmises, celles-ci sont envoyées à un ordinateur externe par liaison informatique, qui peut ainsi éditer les informations sur une imprimante.

L'invention sera mieux comprise à la lumière de la description qui suit, se rapportant à un exemple de réalisation illustratif et en aucun cas limitatif, en référence aux dessins annexés dans lesquels :
- La figure 1 est une représentation schématique de dessus d'un dispositif sous la forme d'une tubulure en liaison directe avec une machine de distribution ou d'aspiration de produit.
- La figure 2 est une représentation schématique de dessus de deux dispositifs sous la forme de tubulures en liaison directe avec une même machine de distribution ou d'aspiration de produit mais avec des applications différentes.
- La figure 3 est une représentation schématique de dessus d'un dispositif sous la forme d'un outil à usage unique en liaison indirecte avec une machine de distribution ou d'aspiration de produit.

L'objet de la présente invention est un dispositif à usage unique, utilisable dans le milieu médical, et plus particulièrement chirurgical.

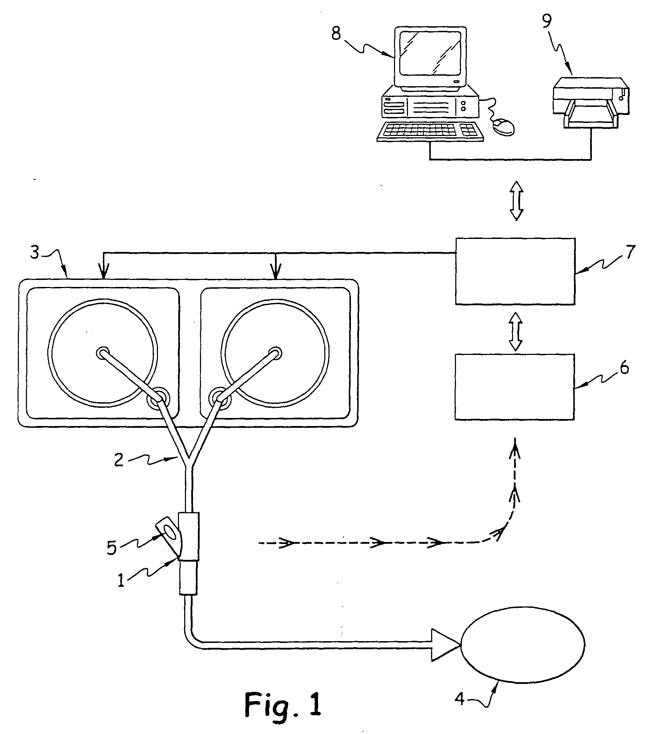

La figure 1 montre une tubulure 1 destinée à être intégrée dans un ensemble tubulaire 2 permettant le passage d'un produit distribué par une machine de distribution de produit 3.

La tubulure 1 est destinée à un usage unique et doit être remplacée à chaque nouvelle utilisation de la machine de distribution de produit 3.

L'ensemble tubulaire 2 relie la machine de distribution de produit 3 à la cavité opératoire 4.

Le patient ou la cavité opératoire 4 reçoit le produit au moyen de l'ensemble tubulaire 2 et de la tubulure 1.

La machine de distribution de produit 3 permet de régler les paramètres liés au produit injecté tels que son débit ou sa pression.

La tubulure 1 intègre un système d'identification par radiofréquences, c'est-à-dire un transpondeur 5.

Le transpondeur 5 se présente selon toute forme connue en soi.

Le transpondeur 5 est ainsi une puce électronique intégrée dans un tube en verre, un composant en matière plastique, une étiquette, etc.

Lors de la fabrication du transpondeur 5 et de son intégration dans la tubulure 1, des données sont préenregistrées dans le transpondeur 5.

Lesdites données préenregistrées se rapportent, par exemple, à la quantité et aux caractéristiques du produit devant être utilisé avec la tubulure 1. En effet, à chaque poche de produit est associée une unique tubulure 1.

Les données préenregistrées concernent également la fonction du produit associé à la tubulure 1, le nom du fabricant, le numéro de lot de fabrication, des informations d'horodatage c'est-à-dire la date de fabrication et de péremption.

De plus, les données préenregistrées se rapportent à des données de réglages spécifiques de la machine de distribution du produit 3 ou d'autres équipements.

Les données préenregistrées sont transmises par radiofréquences à un récepteur 6 de radio fréquences, de type connu en soi.

La transmission des données préenregistrées se fait donc sans contact et sans liaison électrique filaire.

Le récepteur 6 est, soit intégré directement à la machine de distribution du produit 3, soit intégré à un équipement annexe (non représenté ici).

Le récepteur 6 traduit les données préenregistrées reçues et transmet des informations de réglages au gestionnaire électronique 7 de la machine de distribution du produit 3.

Le gestionnaire électronique 7 règle donc la machine de distribution du produit 3 en fonction des informations reçues.

Les informations sur les réglages de la machine de distribution de produit 3 effectués par le gestionnaire électronique 7 sont transmises par liaison informatique de type RS232 ou USB à un ordinateur externe 8 ou à une imprimante 9.

Les informations émises par le récepteur 6 peuvent être également directement transmises à l'ordinateur 8 ou à l'imprimante 9.

Le procédé mis en oeuvre permet ainsi d'enregistrer les données préenregistrées sur un support informatique, de les afficher sur un écran ou de les éditer sur un support papier.

La traçabilité du produit est donc facilement connue et le réglage de ladite machine 3 se fait de manière automatique sans intervention extérieure.

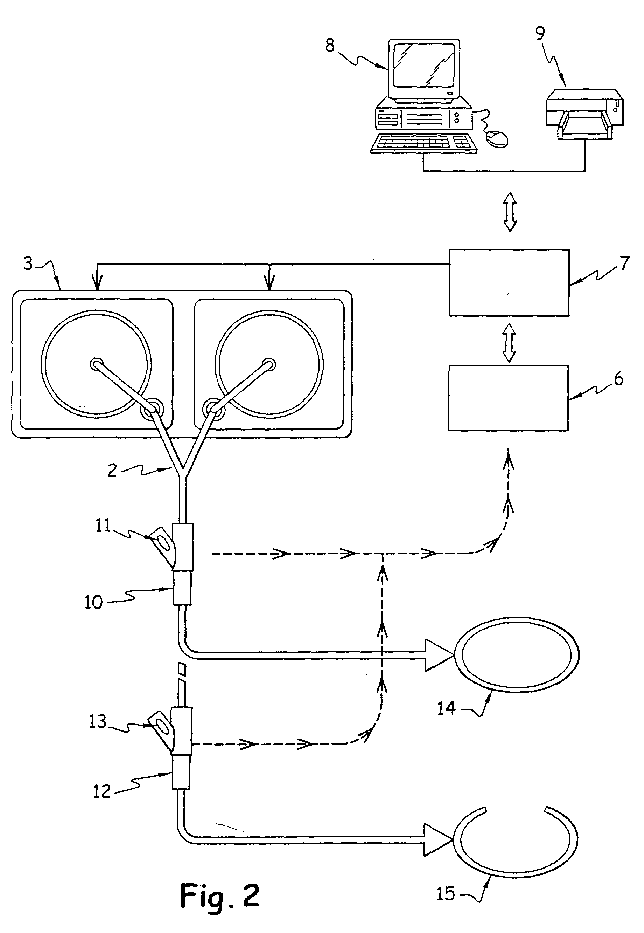

La figure 2 représente une vue schématique d'un ensemble tubulaire 2 relié à une machine de distribution du produit 3 et à une cavité opératoire (14, 15).

La tubulure 10 intégrant le transpondeur 11 permet de faire fonctionner l'ensemble tubulaire 2 dans les conditions requises dans la cavité opératoire 14.

La cavité opératoire 15, différente de la cavité opératoire 14, fonctionne avec le même ensemble tubulaire 2 mais avec la tubulure 12 intégrant le transpondeur 13.

Les tubulures 10 et 12 fonctionnent de la même manière que la tubulure 1.

Elles se différencient par leurs informations préenregistrées permettant de faire fonctionner la machine de distribution du produit 3 selon deux modes de réglages différents.

Le récepteur 6, le gestionnaire électronique 7 ainsi que l'ordinateur 8 et l'imprimante 9 externes sont du même type que ceux de la figure 1.

Les informations préenregistrées dans la tubulure 10 et 12 sont transmises par radiofréquences au récepteur 6, qui transmet ensuite des informations au gestionnaire électronique 7 qui adapte, en fonction de ses informations reçues, le mode de fonctionnement de la machine de distribution du produit 3.

Ainsi, la machine de distribution du produit 3 peut servir pour plusieurs types de chirurgie sans nécessité de réglage manuelle au préalable de ladite machine 3.

La machine adapte son fonctionnement et notamment la pression du produit distribué en fonction de la tubulure (10, 12) connectée à l'ensemble tubulaire 2.

Le dispositif selon l'invention peut également adapter, par exemple, le débit du produit en fonction du type de tubulure.

Ainsi, la cavité opératoire peut aussi bien concerner, avec la même machine de distribution du produit 3, une chirurgie endoscopique qui nécessite une distribution de produit à une pression comprise entre 0 et 350 mbars, qu'une chirurgie à cavité ouverte qui nécessite une pression du produit distribué de 1500 mbars.

Le dispositif selon l'invention permet ainsi de paramétrer, sécuriser et valider le mode de fonctionnement de la machine de distribution du produit en fonction du type de chirurgie pratiquée dans la cavité opératoire et du type de tubulure utilisée.

Les tubulures 10 et 12 peuvent également de manière avantageuse intégrer un capteur (non représenté sur la figure), de type connu en soi, permettant de relever en temps réel ou d'enregistrer des paramètres relatifs au produit circulant dans l'ensemble tubulaire 2 et la machine de distribution du produit 3.

Les paramètres du produit circulant sont, par exemple, les valeurs de la température, de la pression et du débit du produit.

Ces paramètres sont ensuite transmis au transpondeur (11, 13) qui les envoie au récepteur 6.

Le récepteur 6 peut alors envoyer des informations au gestionnaire électronique 7 qui modifie le réglage et le mode de fonctionnement de la machine de distribution du produit 3, si certaines valeurs critiques ou limites sont atteintes, et / ou transmettre les valeurs des paramètres à l'ordinateur externe 8, pour qu'elles soient enregistrées et affichées sur un écran, et à une imprimante 9 pour qu'elles soient éditées.

Le dispositif selon l'invention peut être aussi bien utilisé pour les interventions chirurgicales que pour les systèmes permettant la distribution de liquides ou de médicaments.

Ainsi, dans le domaine de la nutrition, perfusion ou transfusion, des tubulures à usage unique selon l'invention peuvent retranscrire des informations relatives au produit, notamment le nom du fabricant, le numéro de lot, la date de fabrication et de péremption.

Le même dispositif selon l'invention peut également relever s'il y a eu une anomalie dans la distribution du produit par la présence d'un capteur de débit, de pression ou de température.

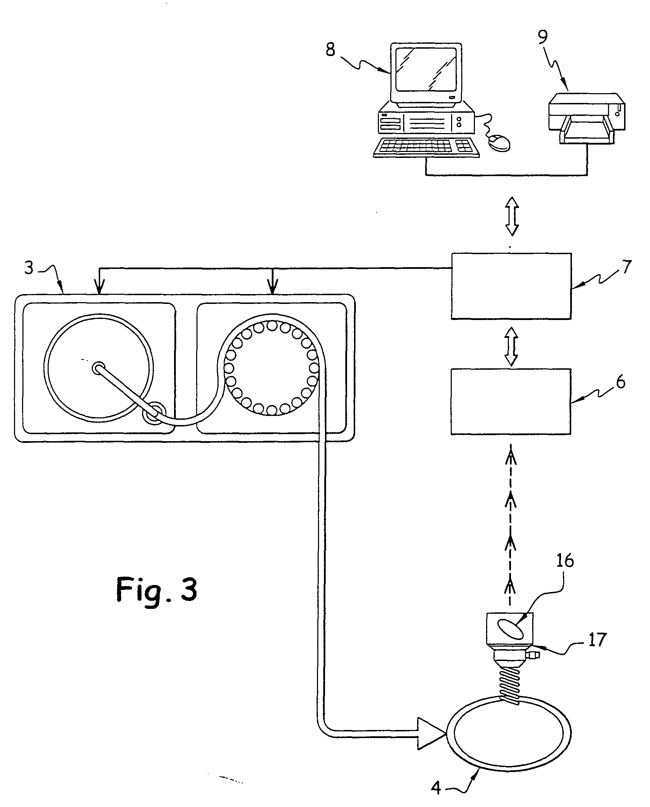

La figure 3 est une autre représentation d'une utilisation possible du dispositif selon l'invention.

La machine de distribution de produit 3, le récepteur 6, le gestionnaire électronique 7 ainsi que l'ordinateur 8 et l'imprimante 9 externes sont du même type que ceux de la figure 1

Le transpondeur 16 est intégré dans un outil 17 à usage unique, non relié par un ensemble tubulaire à la machine de distribution de produit 3.

L'outil est, par exemple, un trocart ou une canule à usage chirurgical.

L'outil 17 fonctionne selon le même principe que les dispositifs à tubulure décrits ci-dessus, c'est-à-dire qu'au transpondeur 16 a été intégré des informations préenregistrées que le transpondeur transmet au récepteur 6, ce qui ensuite permet la modification, en fonction de la nature de ces informations, des réglages de la machine de distribution ou d'aspiration de produit 3.

Le transpondeur 16 peut également recevoir des informations de la machine de distribution du produit 3 lui indiquant des défauts ou des anomalies de ladite machine 3.

L'outil 17 possède un capteur (non représenté) de type connu.

Le capteur permet de mesurer le débit, la pression ou la température du produit circulant ou du fluide circulant dans l'outil 17.

Ainsi, le réglage de la machine de distribution du produit 3 peut être modifié en fonction de la valeur des paramètres mesurés par le capteur de l'outil 17.

Le dispositif selon l'invention de la figure 3 peut ainsi être avantageusement utilisé en chirurgie endoscopique où des outils et/ ou des composants sont introduits dans des cavités opératoires.

De ce fait, le dispositif selon l'invention peut transmettre en temps réel, à l'aide d'un capteur, la pression et la température intra-cavitaire.

## Revendications

1. Dispositif médical intégrant des moyens de transmissions d'informations permettant la gestion des paramètres de distribution / aspiration d'un produit, aux moyens d'une machine de distribution ou d'aspiration de produit (3), dans un patient (4, 14, 15), et un transpondeur (5, 11, 13, 16) émettant lesdites informations par radiofréquence, **caractérisé en ce que** ledit dispositif est à usage unique, **en ce qu'**il permet l'identification d'un produit circulant et **en ce qu'**il consiste en une tubulure (1, 10, 12) reliant ladite machine de distribution ou d'aspiration de produit (3) à un patient (4, 14, 15).

2. Dispositif médical intégrant des moyens de transmissions d'informations permettant la gestion des paramètres de distribution / aspiration d'un produit, aux moyens d'une machine de distribution ou d'aspiration de produit (3), dans un patient (4, 14, 15), et un transpondeur (5, 11, 13, 16) émettant lesdites informations par radiofréquence, **caractérisé en ce que** ledit dispositif est à usage unique, **en ce qu'**il permet l'identification d'un produit circulant et **en ce qu'**il consiste en un outil médical (17), notamment chirurgical.

3. Dispositif selon la revendication 1 ou 2, **caractérisé en ce que** ledit produit est un fluide, notamment un gaz.

4. Dispositif selon l'une des revendications précédentes, **caractérisé en ce que** le transpondeur intègre un capteur permettant de relever en temps réel ou d'enregistrer des paramètres relatifs audit produit circulant, notamment la pression, la température ou le débit dudit produit circulant.

5. Ensemble pour la transmission d'informations comprenant un dispositif médical selon l'une des revendications précédentes, **caractérisé en ce qu'**il comprend, en outre, un récepteur de radiofréquences (6) pour la transmission d'informations.

6. Ensemble selon la revendication 5, **caractérisé en ce qu'**il comprend un gestionnaire électronique (7) de ladite machine auquel sont transmises lesdites informations provenant du récepteur de radiofréquences (6) et qui modifie le réglage du mode de fonctionnement de ladite machine (3) en fonction des informations reçues.

7. Ensemble selon la revendication 5 ou 6, **caractérisé en ce qu'**il comprend un ordinateur externe (8) auquel sont envoyées, par liaison informatique, les informations émises par la transpondeur (5, 11, 13, 16) après leur réception par le récepteur (7).

8. Ensemble selon l'une des revendications 5 à 7, **caractérisé en ce qu'**il comprend une imprimante (9) sur laquelle les informations émises par le transpondeur (5, 11, 13, 16) sont retranscrites.
